# EUROPEAN PATENT SPECIFICATION

(11) **EP 1 539 850 B1**
(45) Date of publication and mention of the grant of the patent: **27.08.2008**
(21) Application number: 03755831.9
(22) Date of filing: 16.09.2003
(51) Int. Cl.: C08G 18/61, C08G 18/62, A61N 1/05

(54) **COMPOUNDS CONTAINING QUATERNARY CARBONS AND SILICON-CONTAINING GROUPS, MEDICAL DEVICES, AND METHODS.**
VERBINDUNGEN DIE QUARTERNÄRE KOHLSTOFFE UND SILIZIUMGRUPPEN ENTHALTEN, MEDIZINSICHE GERÄTE UND VERFAHREN
COMPOSES CONTENANT DES CARBONES QUATERNAIRES, GROUPES CONTENANT UN SILICIUM, DISPOSITIFS MEDICAUX ET PROCEDES

(30) Priority: 17.09.2002 US 411725 P; 29.07.2003 US 490780 P
(43) Date of publication of application: 15.06.2005
(73) Proprietor: MEDTRONIC, INC., Minneapolis, MN 55432 (US)
(72) Inventor: BENZ, Michael, E., Ramsey, MN 55303 (US); HOBOT, Christopher, M., Tonka Bay, MN 55331 (US); SPARER, Randall, V., Andover, MN 55304 (US)
(74) Representative: Hughes, Andrea Michelle
(86) International application number: PCT/US2003/029153
(87) International publication number: WO 2004/026936

(56) References cited:
- EP-A- 0 661 332
- EP-A- 0 940 405
- WO-A-98/50086
- US-B1- 6 420 452

## Description

### Field of the Invention

This invention relates to compounds containing quaternary carbons and silicon-containing groups, preferably such compounds are polymers containing urethane and/or urea groups, particularly elastomers. Such materials are particularly useful as biomaterials in medical devices.

### Background of the Invention

The chemistry of polyurethanes and/or polyureas is extensive and well developed. Typically, polyurethanes and/or polyureas are made by a process in which a polyisocyanate is reacted with a molecule having at least two functional groups reactive with the polyisocyanate, such as a polyol or polyamine. The resulting polymer can be further reacted with a chain extender, such as a diol or diamine, for example. The polyol or polyamine is typically a polyester, polyether, or polycarbonate polyol or polyamine, for example.

Polyurethanes and/or polyureas can be tailored to produce a range of products from soft and flexible to hard and rigid. They can be extruded, injection molded, compression molded, and solution spun, for example. Thus, polyurethanes and polyureas, particularly polyurethanes, are important biomedical polymers, and are used in implantable devices such as artificial hearts, cardiovascular catheters, pacemaker lead insulation, etc.

Commercially available polyurethanes used for implantable applications include BIOSPAN segmented polyurethanes, manufactured by Polymer Technology Group of Berkeley, CA, PELLETHANE segmented polyurethanes, sold by Dow Chemical, Midland, MI, and TECOFLEX segmented polyurethanes sold by Thermedics Polymer Products, Wilmington, MA. Polyurethanes are described in the article "Biomedical Uses of Polyurethanes," by Coury et al., in Advances in Urethane Science and Technology, 9, 130-168, edited by Kurt C. Frisch and Daniel Klempner, Technomic Publishing Co., Lancaster, PA (1984). Typically, polyether polyurethanes exhibit more biostability than polyester polyurethanes and polycarbonate polyurethanes, as these are more susceptible to hydrolysis. Thus, polyether polyurethanes are generally preferred biopolymers.

Polyether polyurethane elastomers, such as PELLETHANE 2363-80A (P80A) and 2363-55D (P55D), which are prepared from polytetramethylene ether glycol (PTMEG) and methylene bis(diisocyanatobenzene) (MDI) extended with 1,4-butanediol (BDO), are widely used for implantable cardiac pacing leads. Pacing leads are electrodes that carry stimuli to tissues and biologic signals back to implanted pulse generators. The use of polyether polyurethane elastomers as insulation on such leads has provided significant advantage over silicone rubber, primarily because of the higher tensile strength of the polyurethanes.

There is some problem, however, with biodegradation of polyether polyurethane insulation, which can cause failure. Polyether polyurethanes are susceptible to oxidation in the body, particularly in areas that are under stress. When oxidized, polyether polyurethane elastomers can lose strength and can form cracks, which might eventually breach the insulation. This, thereby, can allow bodily fluids to enter the lead and form a short between the lead wire and the implantable pulse generator (IPG). It is believed that the ether linkages degrade, perhaps due to metal ion catalyzed oxidative attack at stress points in the material.

One approach to solving this problem has been to coat the conductive wire of the lead. Another approach has been to add an antioxidant to the polyurethane. Yet another approach has been to develop new polyurethanes that are more resistant to oxidative attack. Such polyurethanes include only segments that are resistant to metal induced oxidation, such as hydrocarbon- and carbonate-containing segments. For example, polyurethanes that are substantially free of ether and ester linkages have been developed. This includes the segmented aliphatic polyurethanes of U.S. Pat. No. 4,873,308 (Coury et al.). Another approach has been to include a sacrificial moiety (preferably in the polymer backbone) that preferentially oxidizes relative to all other moieties in the polymer, which upon oxidation provides increased tensile strength relative to the polymer prior to oxidation. This is disclosed in U.S. Pat. Nos. 5,986,034 (DiDomenico et al.), 6,111,052 (DiDomenico et al.), and 6,149,678 (DiDomenico et al.).

Although such materials produce more stable implantable devices than polyether polyurethanes, there is still a need for biostable polymers, particularly polyurethanes suitable for use as insulation on pacing leads.

### Summary of the Invention

The present invention relates to segmented polymers that include diorgano groups having quaternary carbons and silicon-containing groups. The silicon-containing groups are typically silane- and/or siloxane-containing groups. Particularly preferred polymers include urethane groups, urea groups, or combinations thereof (i.e., polyurethanes, polyureas, or polyurethane-ureas). Polymers of the present invention may be random, alternating, block, star block, or combinations thereof. Preferably, the polymer is a segmented polyurethane. Such polymers are preferably used as biomaterials in medical devices. Preferred polymers are also preferably substantially free of ester, ether, and carbonate linkages.

The present invention provides a segmented polymer, and a medical device incorporating such polymer, which includes a soft segment comprising a group of the formula:

-[-(R¹)ₙ-(-Z-(R²)ₘ-)ₚ-(-Si(R)₂-Vᵣ-)ₛ-]_{q}-

wherein: n = 0 or 1; m = 0 or 1; p = 1-100,000; r = 0-100,000; s = 1-100,000; q = 1-100,000; R¹ and R² are each independently a saturated or unsaturated aliphatic group, an aromatic group, or combinations thereof, optionally including heteroatoms (preferably the aromatic groups are within the backbone); Z is -C(R³)₂- wherein each R³ is independently (i.e., may be the same or different) a saturated or unsaturated aliphatic group, an aromatic group, or combinations thereof, optionally including heteroatoms, wherein the two R³ groups within -C(R³)₂- can be optionally joined to form a ring; each R is independently a saturated or unsaturated aliphatic group, an aromatic group, or combinations thereof, optionally including heteroatoms; and V is -O-Si(R)₂- or R¹; with the proviso that the polymer is free of carbonate linkages.

The present invention also provides a segmented polymer, and a medical device that incorporates such polymer, wherein the soft segment of said polymer is prepared from a polymeric starting compound of the formula:

Y-[-(R¹)ₙ-(-Z-(R²)ₘ-)ₚ-(-Si(R)₂-Vᵣ-)ₛ-]_{q}-R⁵-Y

wherein: each Y is independently OH or NR⁴H; n = 0 or 1; m = 0 or 1; p = 1-100,000; r = 0-100,000; s = 1-100,000; q = 1-100,000; R¹, R², and R⁵ are each independently a saturated or unsaturated aliphatic group, an aromatic group, or combinations thereof, optionally including heteroatoms (preferably, the aromatic groups are within the backbone); Z is -C(R³)₂- wherein each R³ is independently a saturated or unsaturated aliphatic group, an aromatic group, or combinations thereof, optionally including heteroatoms, wherein the two R³ groups within -C(R³)₂- can be optionally joined to form a ring; each R is independently a saturated or unsaturated aliphatic group, an aromatic group, or combinations thereof, optionally including heteroatoms; each R⁴ is independently H or a saturated or unsaturated aliphatic group, an aromatic group, or combinations thereof; V is -O-Si(R)₂- or R¹; with the proviso that the polymer is free of carbonate linkages.

It should be understood that in the above formulas, each of the units (e.g., R¹, -Z-(R²)ₘ-, -(-Si(R)₂-Vᵣ-)ₛ-, and V) (if repeated) can vary within any one molecule.

As written, the formulas provided herein (for both the resultant polymers and the polymeric starting materials) encompass alternating, random, block, star block, or combinations thereof (e.g., wherein certain portions of the molecule are alternating and certain portions are random). With respect to star block copolymers, it should be understood that the polymeric segments described herein could form at least a part of one or more atoms of the star, although the segment itself would not necessarily include the core branch point of the star.

Preferably, the polymers, and compounds used to make them, described herein have substantially no tertiary carbons in the main chain (i.e., backbone) of the molecules.

Methods of preparation of such polymers and compounds are also provided.

As used herein, the terms "a," "an," "one or more," and "at least one" are used interchangeably.

As used herein, the term "aliphatic group" means a saturated or unsaturated linear (i.e., straight chain), cyclic, or branched organic hydrocarbon. This term is used to encompass alkyl (e.g., -CH₃, which is considered a "monovalent" group) (or alkylene if within a chain such as -CH₂-, which is considered a "divalent" group), alkenyl (or alkenylene if within a chain), and alkynyl (or alkynylene if within a chain) groups, for example. The term "alkyl group" means a saturated linear or branched hydrocarbon group including, for example, methyl, ethyl, isopropyl, t-butyl, heptyl, dodecyl, octadecyl, amyl, 2-ethylhexyl, and the like. The term "alkenyl group" means an unsaturated, linear or branched hydrocarbon group with one or more carbon-carbon double bonds, such as a vinyl group. The term "alkynyl group" means an unsaturated, linear or branched hydrocarbon group with one or more carbon-carbon triple bonds. The term "aromatic group" or "aryl group" means a mono- or polynuclear aromatic organic hydrocarbon group. These hydrocarbon groups may be substituted with heteroatoms, which can be in the form of functional groups. The term "heteroatom" means an element other than carbon (e.g., nitrogen, oxygen, sulfur, chlorine, etc.).

As used herein, a "biomaterial" may be defined as a material that is substantially insoluble in body fluids and tissues and that is designed and constructed to be placed in or onto the body or to contact fluid or tissue of the body. Ideally, a biomaterial will not induce undesirable reactions in the body such as blood clotting, tissue death, tumor formation, allergic reaction, foreign body reaction (rejection) or inflammatory reaction; will have the physical properties such as strength, elasticity, permeability and flexibility required to function for the intended purpose; can be purified, fabricated and sterilized easily; and will substantially maintain its physical properties and function during the time that it remains implanted in or in contact with the body. A "biostable" material is one that is not broken down by the body, whereas a "biocompatible" material is one that is not rejected by the body.

As used herein, a "medical device" may be defined as a device that has surfaces that contact blood or other bodily tissues in the course of their operation. This can include, for example, extracorporeal devices for use in surgery such as blood oxygenators, blood pumps, blood sensors, tubing used to carry blood and the like which contact blood which is then returned to the patient. This can also include implantable devices such as vascular grafts, stents, electrical stimulation leads, heart valves, orthopedic devices, catheters, shunts, sensors, replacement devices for nucleus pulposus, cochlear or middle ear implants, intraocular lenses, and the like.

### Brief Description of the Drawing

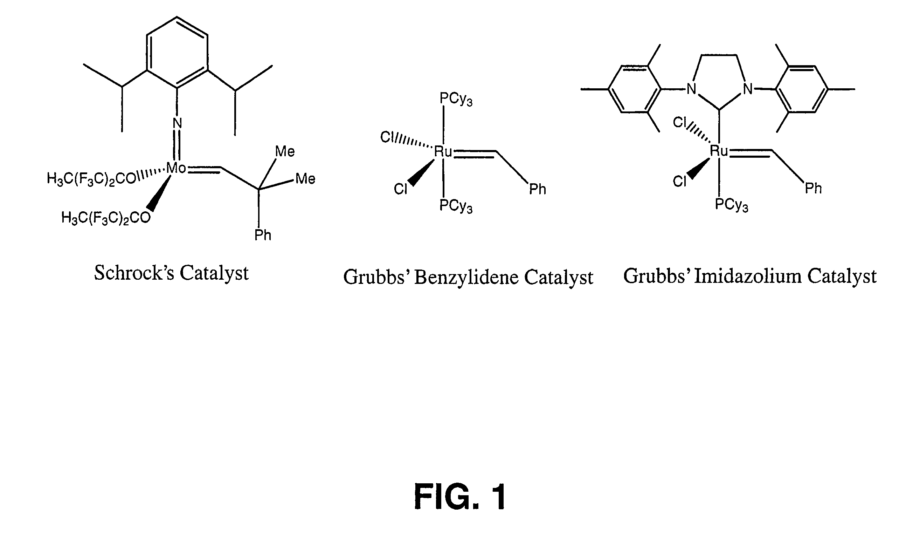

Figure 1 lists examples of catalysts suitable for use in methods of the invention.

### Detailed Description of Illustrative Embodiments of the Invention

The present invention provides segmented polymers (preferably, segmented polyurethanes), compounds used to prepare the soft segments of segmented polymers, and medical devices that include such polymers (preferably, biomaterials). Preferably, the polymers are generally resistant to oxidation and/or hydrolysis, particularly with respect to their backbones, as opposed to their side chains.

The polymers include one or more diorgano groups. These diorgano (e.g., gem-dialkyl) groups are of the general formula -C(R³)₂- wherein C is a quaternary carbon and each R³ is independently (i.e., may be the same or different) a saturated or unsaturated aliphatic group, an aromatic group, or combinations thereof, optionally including heteroatoms (which may be in the chain of the organic group or pendant therefrom as in a functional group). Preferably, each R³ is independently a straight chain alkyl group, optionally including heteroatoms. Most preferably, each R³ is independently a straight chain alkyl group without heteroatoms.

The polymers also include one or more silicon-containing groups. These silicon-containing groups are of the formula -Si(R)₂-Vᵣ- wherein V is of the formula -O-Si(R)₂- (thereby forming a siloxane group) or is R¹ (thereby forming a silane group). Each R is independently a saturated or unsaturated aliphatic group, an aromatic group, or combinations thereof, optionally including heteroatoms (which may be in the chain of the organic group or pendant therefrom as in a functional group). Each R¹ is independently a saturated or unsaturated aliphatic group, an aromatic group, or combinations thereof, optionally including heteroatoms (which may be in the chain of the organic group or pendant therefrom as in a functional group). Preferably, the aromatic groups of R¹ are within the backbone of the polymer chain.

Polymers of the present invention can be used in medical devices as well as nonmedical devices. Preferably, they are used in medical devices and are suitable as biomaterials. Examples of medical devices are listed above. Examples of nonmedical devices include foams, insulation, clothing, footwear, paints, coatings, adhesives, building construction materials, etc.

The polymers suitable for forming biomaterials for use in medical devices according to the present invention include quaternary carbons, silicon-containing groups, and are preferably polyurethanes, polyureas, or polyurethane-ureas. These polymers can vary from hard and rigid to soft and flexible. Preferably, the polymers are elastomers. An "elastomer" is a polymer that is capable of being stretched to approximately twice its original length and retracting to approximately its original length upon release.

Polymers of the present invention can be random, alternating, block, star block, or combinations thereof. Most preferably, the polymers are segmented copolymers (i.e., containing both hard and soft domains or segments) which are comprised substantially of alternating relatively soft segments and relatively hard segments.

For segmented polymers, either the soft segments, or both the hard and the soft segments, include a diorgano moiety and a silicon-containing moiety, thereby providing a polymer that has reduced susceptibility to oxidation and/or hydrolysis, at least with respect to the polymer backbone. As used herein, a "hard" segment is one that is either crystalline at use temperature or amorphous with a glass transition temperature above use temperature (i.e., glassy), and a "soft" segment is one that is amorphous with a glass transition temperature below use temperature (i.e., rubbery). A crystalline or glassy moiety or hard segment is one that adds considerable strength and higher modulus to the polymer. Similarly, a rubbery moiety or soft segment is one that adds flexibility and lower modulus, but may add strength particularly if it undergoes strain crystallization, for example. The random or alternating soft and hard segments are linked by urethane and/or urea groups and the polymers may be terminated by hydroxyl, amine, and/or isocyanate groups.

As used herein, a "crystalline" material or segment is one that has ordered domains. A "noncrystalline" material or segment is one that is amorphous (a noncrystalline material may be glassy or rubbery). A "strain crystallizing" material is one that forms ordered domains when a strain or mechanical force is applied.

An example of a medical device for which the polymers are particularly well suited is a medical electrical lead, such as a cardiac pacing lead, a neurostimulation lead, etc. Examples of such leads are disclosed, for example, in U.S. Pat. Nos. 5,040,544 (Lessar et al.), 5,375,609 (Molacek et al.), 5,480,421 (Otten), and 5,238,006 (Markowitz).

### Polymers and Methods of Preparation

A wide variety of segmented polymers are provided by the present invention. They can be random, alternating, block, star block (or combinations thereof), preferably they are copolymers (including terpolymers, tetrapolymers), that can include olefins, amides, esters, imides, epoxies, ureas, urethanes, carbonates, sulfones, ethers, acetals, phosphonates, and the like. These include moieties containing diorgano (preferably, gem-dialkyl) groups of the general formula -C(R³)₂- wherein C is a quaternary carbon.

Such polymers can be prepared using a variety of techniques from polymerizable compounds (e.g., monomers, oligomers, or polymers) containing diorgano (preferably, gem-dialkyl) moieties of the general formula -C(R³)₂- wherein C is a quaternary carbon, and/or silicon-containing moieties of the formula -Si(R)₂-R¹ᵣ- (thereby forming a silane group) or -Si(R)₂-(O-Si(R)₂)ᵣ- (thereby forming a siloxane group). Such compounds include dienes, diols, diamines, or combinations thereof, for example.

Although certain preferred polymers are described herein, the polymers used to form the preferred biomaterials in the medical devices of the present invention can be a wide variety of polymers that include urethane groups, urea groups, or combinations thereof. Such polymers are prepared from isocyanate-containing compounds, such as polyisocyanates (preferably diisocyanates) and compounds having at least two functional groups reactive with the isocyanate groups, such as polyols and/or polyamines (preferably diols and/or diamines). Any of these reactants can include a diorgano and/or silicon-containing moiety (preferably in the polymer backbone), although preferably a diorgano and/or silicon-containing moiety is provided by the polyols and/or polyamines, particularly diols and/or diamines (including the diols or diamines of the dimer acid described below).

The presence of the diorgano moiety and silicon-containing moiety provides a polymer that is more resistant to oxidative and/or hydrolytic degradation but still has a relatively low glass transition temperature (Tg). Furthermore, preferably, both the hard and soft segments are themselves substantially ether-free, ester-free, and carbonate-free polyurethanes, polyureas, or combinations thereof.

Preferred polymers of the present invention include a group of the formula -(R¹)ₙ-(-Z-(R²)ₘ-)ₚ-, wherein -Z- is a diorgano moiety -C(R³)₂-, and a group of the formula -(-Si(R)₂-Vᵣ-)ₛ- wherein V is of the formula -O-Si(R)₂- (thereby forming a siloxane group) or is R¹ (thereby forming a silane group). In one embodiment, particularly preferred polymers also include one or more urethane groups, urea groups, or combinations thereof (preferably, just urethane groups). In another embodiment, particularly preferred polymers are copolymers (i.e., prepared from two or more monomers, including terpolymers or tetrapolymers). Thus, the present invention provides polymers with these groups randomly distributed or ordered in blocks or segments.

Polymers of the present invention can be linear, branched, or crosslinked. This can be done using polyfunctional isocyanates or polyols (e.g., diols, triols, etc.) or using compounds having unsaturation or other functional groups (e.g., thiols) in one or more monomers with radiation crosslinking, for example. Such methods are well known to those of skill in the art.

Preferably, such polymers (and the compounds used to make them) have substantially no tertiary carbons in the main chain (i.e., backbone).

In the quaternary-carbon group of the formula -(R¹)ₙ-(-Z-(R²)ₘ-)ₚ-, n = 0 or 1; m = 0 or 1; p = 1-100,000; R¹ and R² are each independently a saturated or unsaturated aliphatic group, an aromatic group, or combinations thereof, optionally including heteroatoms (which may be in the chain of the organic group or pendant therefrom as in a functional group), preferably with the proviso that the aromatic groups are within the backbone; and Z is a diorgano moiety -C(R³)₂- wherein each R³ is independently a saturated or unsaturated aliphatic group, an aromatic group, or combinations thereof, optionally including heteroatoms (which may be in the chain of the organic group or pendant therefrom as in a functional group), wherein the two R³ groups within a -C(R³)₂- moiety can be optionally joined to form a ring. It should be understood that the repeat units -Z-(R²)ₘ- can vary within any one molecule.

In the silicon-containing group of the formula -Si(R)₂-Vᵣ-, r = 0-100,000; s = 1-100,000, V is of the formula -O-Si(R)₂- (thereby forming a siloxane group) or is R¹ (thereby forming a silane group). Each R is independently a saturated or unsaturated aliphatic group, an aromatic group, or combinations thereof, optionally including heteroatoms (which may be in the chain of the organic group or pendant therefrom as in a functional group). Each R¹ group is as defined above.

Preferred polymers include a group of the formula

-[-(R¹)ₙ-(-Z-(R²)ₘ-)ₚ-(-Si(R)₂-Vᵣ-)ₛ-]_{q}-

wherein each of the numerical variables (n, m, p, r, and s) and the organic groups (R, R¹, and R²) and the V group are as defined above. In this formula q = 1-100,000.

A preferred source of the group of the formula

-[-(R¹)ₙ-(-Z-(R²)ₘ-)ₚ-(-Si(R)₂-Vᵣ-)ₛ-]_{q}-

is a compound (typically a polymeric starting compound) of the formula (Formula I):

Y-(R¹)ₙ-(-Z-(R²)ₘ-)ₚ-(-Si(R)₂-Vᵣ-)ₛ-]_{q}-R⁵-Y

wherein: each Y is independently OH or NR⁴H; n = 0 or 1; m = 0 or 1; p = 1-100,000; r = 0-100,000; s = 1-100,000; q = 1-100,000 (preferably q = 1); R¹, R², and R⁵ are each independently a saturated or unsaturated aliphatic group, an aromatic group, or combinations thereof, optionally including heteroatoms, preferably with the proviso that the aromatic groups are within the backbone; Z is a diorgano moiety -C(R³)₂- wherein each R³ is independently a saturated or unsaturated aliphatic group, an aromatic group, or combinations thereof, optionally including heteroatoms, wherein the two R³ groups within a -C(R³)₂- moiety can be optionally joined to form a ring; each R is independently a saturated or unsaturated aliphatic group, an aromatic group, or combinations thereof, optionally including heteroatoms; each R⁴ is independently H or a saturated or unsaturated aliphatic group, an aromatic group, or combinations thereof; and V is -O-Si(R)₂- or R¹.

It should be understood that any repeat unit can vary within any one molecule. For example, in addition to each R being the same or different within each Si(R)₂ group, the Si(R)₂ groups (if repeated) can be the same or different in any one molecule. Also, in addition to each R² being the same or different within each Z(R²)ₘ group, the Z(R²)ₘ groups (if repeated) can be the same or different in any one molecule. Furthermore, each R¹ (if repeated) can be the same or different in any one molecule.

The R³ groups on the quaternary carbon (in the Z groups) are preferably selected such that the ultimate product (e.g., a segmented polyurethane polymer) has one or more of the following properties (preferably, all of the following properties) relative to a polymer without the diorgano (Z) moieties: reduced glass transition temperature (Tg) of the polymer; enhanced strength as a result of hydrogen bonding between the polymer chains; suppressed crystallization of soft segments at room temperature under zero strain; increased strain crystallization; greater ability to control phase separation for balancing elastomeric properties versus strength; greater ability to control melt rheology; and greater ability to modify the polymers using functional groups within the R³ groups.

Although the diorgano moieties reduce the susceptibility of the compound of Formula I and the ultimate polymer to oxidation or hydrolysis, the R³ groups could themselves be susceptible to oxidation or hydrolysis as long as the main chain (i.e., the backbone) is not generally susceptible to such reactions. Preferably, the R³ groups are each independently a straight chain alkyl group, an aryl group, or combinations thereof. More preferably, the R³ groups are each independently a straight chain alkyl group.

Optionally, the R³ groups can include heteroatoms, such as nitrogen, oxygen, phosphorus, sulfur, and halogen. These could be in the chain of the organic group or pendant therefrom in the form of functional groups, as long as the polymer is generally resistant to oxidation and/or hydrolysis, particularly with respect to its backbone, as opposed to its side chains. Such functional groups include, for example, an alcohol, ether, acetoxy, ester, aldehyde, acrylate, amine, amide, imine, imide, and nitrile, whether they be protected or unprotected. Most preferably, each R³ is independently a straight chain alkyl group without heteroatoms.

The R groups on the silicon atoms are preferably selected such that the ultimate product (e.g., a segmented polyurethane polymer) has one or more of the following properties (preferably, all of the following properties) relative to a polymer without the silicon-containing moieties: greater chain flexibility; less susceptibility to oxidation and hydrolysis; and greater ability to modify the polymers using functional groups within the R groups.

Although the silicon-containing moieties reduce the susceptibility of the compound of Formula I and the ultimate polymer to oxidation or hydrolysis, the R groups could themselves be susceptible to oxidation or hydrolysis as long as the main chain (i.e., backbone) is not generally susceptible to such reactions. Preferably, the R groups are each independently a straight, branched, or cyclic alkyl or alkenyl group, a phenyl group, or a straight chain or branched alkyl substituted phenyl group. More preferably, each R group is a straight chain alkyl group.

Optionally, the R groups can include heteroatoms, such as nitrogen, oxygen, phosphorus, sulfur, and halogen. These could be in the chain of the organic group or pendant therefrom in the form of functional groups, as long as the polymer is generally resistant to oxidation and/or hydrolysis, particularly with respect to its backbone, as opposed to its side chains. Such functional groups include, for example, an alcohol, ether, acetoxy, ester, aldehyde, acrylate, amine, amide, imine, imide, and nitrile, whether they be protected or unprotected. Most preferably, each R is independently a straight chain alkyl group without heteroatoms.

Preferably, R¹ and R² are each independently a straight chain alkylene group (e.g., a divalent aliphatic group such as -CH₂-CH₂- and the like), an arylene group, or combinations thereof, preferably with the proviso that the aromatic groups are within the backbone. More preferably, R¹ and R² do not include tertiary carbon atoms in the main chain (i.e., backbone) of the molecule. Most preferably, R¹ and R² are each independently a straight chain alkylene group.

Preferably, each R⁴ group is independently hydrogen, a straight chain alkyl group, an aryl group, or combinations thereof. More preferably, each R⁴ group is independently hydrogen or a straight chain alkyl group.

The R, R¹, R², R³, and R⁴ groups are selected such that the number average molecular weight of a compound of Formula I is no greater than about 100,000 grams per mole (g/mol or Daltons). Preferably, the molecular weight is about 1000 g/mol to about 1500 g/mol.

Preferably, R, R¹, and R² are each independently an organic group that includes at least one carbon atom, and more preferably at least two carbon atoms. Preferably, R, R¹, and R² are each independently an organic group that includes no more than (i.e., up to) 100 carbon atoms, more preferably no more than 50 carbon atoms, and most preferably no more than 20 carbon atoms.

Preferably, R³ is an organic group that includes at least one carbon atom. Preferably, R³ is an organic group that includes no more than 100 carbon atoms, more preferably no more than 50 carbon atoms, and most preferably no more than 20 carbon atoms.

Preferably, R⁴ is hydrogen or an organic group that includes at least one carbon atom. Preferably, if R⁴ is an organic group, it includes no more than 100 carbon atoms, more preferably no more than 50 carbon atoms, even more preferably no more than 20 carbon atoms, and most preferably no more than 4 carbon atoms. Most preferably, R⁴ is hydrogen.

The values for n, m, p, r, s, and q are average values. Preferably, at least one n or m is one. More preferably, both n and m are one. In increasing order of preference, p, s, and q are each independently 1-100,000, 1-50,000, 1-10,000, 1-5000, 1-2000, 1-1000, 1-500, 1-200, 1-100, 1-50, 1-20, 2-20, and 2-12. Preferably, q = 1 for the starting compound of Formula I. In increasing order of preference, r is 0-100,000, 0-200, and 0-20.

Preferably, the Y groups are OH of NH₂. More preferably, the Y groups are both OH.

The polymers of the present invention can be prepared using standard techniques. Certain polymers can be made using one or more of the compounds of Formula I. Typically, a compound of Formula I is combined with an organic compound containing two or more groups capable of reacting with hydroxyl or amine groups.

For example, if Y in Formula I is an amine (NR⁴H), one could react those amines with di-, tri- or poly(acids), di-, tri, or poly(acyl chlorides), or with cyclic amides (lactams) to form poly(amides). Alternatively, one could react those amines with di-, tri- or poly(anhydrides) to make poly(imides). Alternatively, one could react those amines with glycidyl-containing compounds to form epoxies.

If Y in Formula I is hydroxyl (OH), one could react those hydroxyl groups with di-, tri-, or poly(acids), di-, tri-, or poly(acyl chlorides), or with cyclic esters (lactones) to form poly(esters). Alternatively, one could react those hydroxyl groups with vinyl ether-containing compounds to make poly(acetals). Alternatively, one could react those hydroxyls with sodium hydroxide to form sodium salts, and further react those salts with phosgene to form poly(carbonates). Reacting those sodium salts with other alkyl halide containing moieties can lead to poly(sulfones) and poly(phosphates) and poly(phosphonates).

Typically, the preferred urethane- and/or urea-containing polymers are made using polyisocyanates and one or more compounds of Formula I. It should be understood, however, that diols or diamines that do not contain such diorgano or silicon-containing moieties can also be used to prepare the urethane- and/or urea-containing polymers of the present invention, as long as the resultant polymer includes at least some diorgano and silicon-containing moieties either from diols or diamines or other reactants. Also, other polyols and/or polyamines can be used, including polyester and polyether polyols, for example, although such polyols are less preferred because they produce less biostable materials. Furthermore, the polyols and polyamines can be aliphatic, cycloaliphatic, aromatic, heterocyclic, or combinations thereof.

Examples of suitable polyols (typically diols) include those commercially available under the trade designation POLYMEG and other polyethers such as polyethylene glycol and polypropylene oxide, polybutadiene diol, dimer diol (e.g., that commercially available under the trade designation DIMEROL (Unichema North America of Chicago, IL), and polyester-based diols such as those commercially available from STEPANPOL (from Stepan Corp., Northfield, IL), CAPA (a polycaprolactone diol from Solvay, Warrington, Cheshire, United Kingdom), TERATE (from Kosa, Houston, TX), poly(ethylene adipate) diol, poly(ethylene succinate) diol, poly(1,4-butanediol adipate) diol, poly(caprolactone) diol, poly(hexamethylene phthalate) diol, and poly(1,6-hexamethylene adipate) diol.

Other polyols can be used as chain extenders in the preparation of polymers, as is conventionally done in the preparation of polyurethanes, for example. Examples of suitable chain extenders include 1,10-decanediol, 1,12-dodecanediol, 9-hydroxymethyl octadecanol, cyclohexane-1,4-diol, cyclohexane-1,4-bis(methanol), cyclohexane-1,2-bis(methanol), ethylene glycol, diethylene glycol, 1,3-propylene glycol, dipropylene glycol, 1,2-propylene glycol, trimethylene glycol, 1,2-butylene glycol, 1,3-butanediol, 2,3-butanediol, 1,4-butanediol, 1,5-pentanediol, 1,6-hexanediol,1,2-hexylene glycol, 1,2-cyclohexanediol, 2-butene-1,4-diol, 1,4-cyclohexanedimethanol, 2,4-dimethyl-2,4-pentanediol, 2-methyl-2,4-pentanediol, 1,2,4-butanetriol, 2-ethyl-2-(hydroxymethyl)-1,3-propanediol, glycerol, 2-(hydroxymethyl)-1,3-propanediol, neopentyl glycol, pentaerythritol, and the like.

Examples of suitable polyamines (typically diamines) include ethylenediamine, 1,4-diaminobutane, 1,10-diaminodecane, 1,12-diaminododecane, 1,8-diaminooctane, 1,2-diaminopropane, 1,3-diaminopropane, tris(2-aminoethyl)amine, lysine ethyl ester, and the like.

Examples of suitable mixed alcohols/amines include 5-amino-1-pentanol, 6-amino-1-hexanol, 4-amino-1-butanol, 4-aminophenethyl alcohol, ethanolamine, and the like.

Suitable isocyanate-containing compounds for preparation of polyurethanes, polyureas, or polyurethanes-ureas, are typically aliphatic, cycloaliphatic, aromatic, and heterocyclic (or combinations thereof) polyisocyanates. In addition to the isocyanate groups they can include other functional groups such as biuret, urea, allophanate, uretidine dione (i.e., isocyanate dimer), and isocyanurate, etc., that are typically used in biomaterials. Suitable examples of polyisocyanates include 4,4'-diisocyanatodiphenyl methane (MDI), 4,4'-diisocyanatodicyclohexyl methane (HMDI), cyclohexane-1,4-diisocyanate, cyclohexane-1,2-diisocyanate, isophorone diisocyanate, tolylene diisocyanates, naphthylene diisocyanates, benzene-1,4-diisocyanate, xylene diisocyanates, trans-1,4-cyclohexylene diisocyanate, 1,4-diisocyanatobutane, 1,12-diisocyanatododecane, 1,6-diisocyanatohexane, 1,5-diisocyanato-2-methylpentane, 4,4'-methylenebis(cyclohexyl isocyanate), 4,4'- methylenebis(2,6-diethyphenyl isocyanate), 4,4'-methylenebis(phenyl isocyanate), 1,3-phenylene diisocyanate, poly((phenyl isocyanate)-co-formaldehyde), tolylene-2,4-diisocyanate, tolylene-2,6-diisocyanate, dimer diisocyanate, as well as polyisocyanates available under the trade designations DESMODUR RC, DESMODUR RE, DESMODUR RFE, and DESMODUR RN from Bayer, and the like.

The relatively hard segments of the polymers of the present invention are preferably fabricated from short to medium chain diisocyanates and short to medium chain diols or diamines, all of which preferably have molecular weights of less than about 1000 g/mol. Appropriate short to medium chain diols, diamines, and diisocyanates include straight chain, branched, and cyclic aliphatics, although aromatics can also be used. Examples of diols and diamines useful in these more rigid segments include both the short and medium chain diols or diamines discussed above.

In addition to the polymers described herein, biomaterials of the invention can also include a variety of additives. These include, antioxidants, colorants, processing lubricants, stabilizers, imaging enhancers, fillers, and the like.

### Starting Materials and Methods of Preparation

The compounds of Formula I above can be made by the synthetic route described in the Examples Section. The method typically includes combining monomers of Formula II

R¹⁰HC=CH-(R¹¹)_{r'}-(-Si(R)₂-Vᵣ-)ₛ-(R¹²)_{s'}-CH=CHR¹³

or Formula III

R¹⁰HC=CH-(R¹¹)_{r'}-Z-(R¹²)_{s'}-CH=CHR¹³

(which are described in greater detail below) with an alkene metathesis catalyst and optionally applying a vacuum. This typically involves an intermediate in which Y is a protected group such as an acetoxy (-OC(O)CH₃), a benzyl ether (-OCH₂phenyl), a tertiary butyl carbamate (-NR⁴-C(O)-t-butyl), or a benzyl carbamate (-NR⁴-C(O)OCH₂phenyl).

Thus, a preferred polymeric starting compound is that of the formula (Formula I):

Y-[-(R¹)ₙ-(-Z-(R²)ₘ-)ₚ-(-Si(R)₂-Vᵣ-)ₛ-]_{q}-R⁵-Y

as described above.

Preferably, in the compound of the Formula I: each Y is independently OH; each of the R groups are straight chain saturated alkyls or alkylenes having 1-20 carbon atoms; and each of p, s, and q are 1-50.

Such compounds can be made starting with a diene compound having a quaternary carbon (i.e., a diorgano group referred to herein as Z or a -C(R³)₂- group), a diene with a silicon-containing compound, a chain transfer agent, and optionally a chain extender. The dienes could be the same compounds, such that one diene includes both quaternary carbon and silicon. The diene compounds are polymerized, optionally with a chain extender, in the presence of an ADMET (Acyclic Diene Metathesis) catalyst followed by incorporation of a chain transfer agent yielding an unsaturated telechelic polymer.

The two carbon-carbon double bonds of the diene compound can be either internal or terminal as long as they are separated by the Z group.

Preferably, the diene with the silicon is a compound of the formula (Formula II):

R¹⁰HC=CH-(R¹¹)_{r'}-(-Si(R)₂-Vᵣ-)ₛ-(R¹²)_{s'}-CH=CHR¹³

and the diene with the quaternary carbon is a compound of the formula (Formula III):

R¹⁰HC=CH-(R¹¹)_{r'}-Z-(R¹²)_{s'}-CH=CHR¹³

wherein: r, s, V, and R are as defined above; r' = 0 or 1; s' = 0 or 1; Z is a -C(R³)₂- group as defined above; R¹⁰ and R¹³ are each independently hydrogen or straight chain, branched, or cyclic alkyl groups containing up to 6 carbon atoms; and R¹¹ and R¹² are each independently a saturated aliphatic group, an aromatic group, or combinations thereof, preferably with the proviso that the aromatic groups are within the chain. Preferably, using this synthetic procedure R³ does not include unsaturated aliphatic groups, although it can include aromatic groups. The resultant polymers, however, could be subsequently modified to include aliphatic unsaturation.

Preferably, R¹¹ and R¹² are each independently a straight chain alkylene group, an arylene group, or combinations thereof, preferably with the proviso that the aromatic groups are within the chain. More preferably, R¹¹ and R¹² are each independently a straight chain alkylene group. Preferably, R¹¹ and R¹² are each independently an organic group that includes at least one carbon atom, and more preferably at least two carbon atoms. Preferably, R¹¹ and R¹² are each independently an organic group that includes no more than 100 carbon atoms, more preferably no more than 50 carbon atoms, and most preferably no more than 20 carbon atoms. Preferably, at least one of r' or s' is one. More preferably, both r' and s' are one.

A chain extender can be optionally used to alter the spacing between the Z groups in the resultant polymer. This also has the added advantage of allowing for a broader range of glass transition temperatures (Tg's) than can normally be realized upon polymerizing one monomer. The chain extender is a diene wherein the two carbon-carbon double bonds are either internal or terminal. Preferably, it is a compound of the formula (Formula IV):

R¹⁴HC=CH-R¹⁵-CH=CHR¹⁶

wherein: R¹⁴ and R¹⁶ are each independently hydrogen or straight chain, branched, or cyclic alkyl groups containing up to 6 carbon atoms; and R¹⁵ is a saturated aliphatic group, an aromatic group, or combinations thereof, preferably with the proviso that the aromatic groups are within the chain.

Preferably, R¹⁵ is a straight chain alkylene group, an arylene group, or combinations thereof, preferably with the proviso that the aromatic groups are within the chain. More preferably, R¹⁵ is a straight chain alkylene group. Preferably, R¹⁵ is an organic group that includes at least one carbon atom, and more preferably at least two carbon atoms. Preferably, R¹⁵ is an organic group that includes no more than 100 carbon atoms, more preferably no more than 50 carbon atoms, and most preferably no more than 20 carbon atoms.

The chain transfer agent includes protecting groups and is preferably a compound of the formula (Formula V):

Y-R¹⁷-HC=CH-R¹⁸-Y

wherein: each Y is independently a protected form of an OH or an NR⁴H group (e.g., wherein Y is an acetoxy, a benzyl ether, a tertiary butyl carbamate, or a benzyl carbamate); R¹⁷ and R¹⁸ are each independently a saturated aliphatic group, an aromatic group, or combinations thereof, preferably with the proviso that the aromatic groups are within the chain.

Preferably, R¹⁷ and R¹⁸ are each independently a straight chain alkylene group, an arylene group, or combinations thereof, preferably with the proviso that the aromatic groups are within the chain. More preferably, R¹⁷ and R¹⁸ are each independently a straight chain alkylene group. Preferably, R¹⁷ and R¹⁸ are each independently an organic group that includes at least one carbon atom, and more preferably at least two carbon atoms. Preferably, R¹⁷ and R¹⁸ are each independently an organic group that includes no more than 100 carbon atoms, more preferably no more than 50 carbon atoms, and most preferably no more than 20 carbon atoms.

Alternatively, the chain transfer agent can include one alkene group and only one protected alcohol or amine. The alkene can be terminal or, if not terminal, it can include a relatively small alkyl substituent that forms a volatile compound under the metathesis conditions. An example of this type of chain transfer agent is 10-undecene-1-yl-acetate. Such a compound is generally of the formula (Formula VI):

R¹⁹-HC=CH-R²⁰-Y

wherein: Y is a protected form of an OH or an NR⁴H group (e.g., wherein Y is an acetoxy, a benzyl ether, a tertiary butyl carbamate, or a benzyl carbamate); R¹⁹ and R²⁰ are each independently a saturated aliphatic group, an aromatic group, or combinations thereof, preferably with the proviso that the aromatic groups are within the chain; R¹⁹ can also be hydrogen. Preferably, R¹⁹ is a (C1-C6)alkyl group, and more preferably R¹⁹ is H. If a compound of Formula VI is reacted with a compound of Formula III, the metathetic by-product would be of the formula R¹⁰HC=CHR¹⁹, which should have sufficiently small R¹⁰ and R¹⁹ groups to be volatile under the conditions of the polymerization reaction.

The ADMET catalyst can be any of a variety of catalysts capable of effecting metathesis polymerization. Examples include Schrock's molybdenum alkylidene catalyst, Grubbs' ruthenium benzylidene catalyst, and Grubbs' imidazolium catalyst, as shown in Figure 1, which are well known to those of skill in the art.

Preferably, the quaternary carbon-containing and silicon-containing diene compound(s) are combined with an ADMET catalyst under conditions effective to cause polymerization to a high molecular weight intermediate (e.g., a number average molecular weight of about 10,000 g/mol to about 1 x 10⁶ g/mol). Optionally, a chain extender can be added before the catalyst is added. Typically, conditions of this polymerization include reduced pressure (e.g., less than about 10 milliTorr (1.33 pascals)) at a temperature of about 0°C to about 100°C (preferably, about 25°C to about 60°C) and a time of about 1 hour to about 10 days (preferably, about 48 hours to about 120 hours). The reduced pressure is desired to remove metathetic by-products and reduce the number of terminal olefins. This high molecular weight intermediate can be stored for later reaction if desired.

This high molecular weight intermediate is then combined with a chain transfer agent in the presence of the same or a different ADMET catalyst under conditions effective to depolymerize the high molecular weight intermediate and form an unsaturated telechelic polymer. Typically, such conditions include an inert atmosphere (e.g., argon) or under reduced pressure (e.g., less than about 10 milliTorr (1.32 x 10⁻⁵ atmospheres or 1.33 pascals (Pa)) and a temperature of about 0°C to about 100°C (preferably, about 50°C to 60°C) and a time of about 1 hour to about 10 days (preferably, about 24 hours to about 96 hours). The amount of chain transfer agent controls the molecular weight of the unsaturated telechelic polymer. Optionally, this depolymerization reaction is carried out in an organic solvent (e.g., toluene) to reduce the viscosity.

Optionally, the unsaturated telechelic polymer could be formed in a one-step reaction in which the quaternary carbon-containing and silicon-containing diene compound(s), optional chain extender, and a chain transfer agent are combined prior to the addition of the ADMET catalyst to the mixture. This may or may not be carried out in an organic solvent.

The unsaturated telechelic polymer is then subjected to a hydrogenation reaction. This is preferably carried out in the presence of a hydrogenation catalyst under conditions effective to form a fully saturated telechelic polymer. The hydrogenation catalyst is preferably palladium on activated carbon, but could be others well known in the art. Typically, such conditions include the use of a hydrogen pressure of about 1 psig (0.068 atmospheres, 6.89 Pa) to about 1000 psig (68 atmospheres, 6.89 MPa) (preferably, about 300 psig (20 atmospheres, 2.03 MPa) to about 500 psig (34 atmospheres, 3.45 MPa)) and a temperature of about 0°C to about 200°C (preferably, about 60°C to about 100°C) and a time of about 1 hour to about 10 days (preferably, about 3 days to about 5 days).

Alternatively, the hydrogenation reaction can be carried out using para-toluenesulfonhydrazide in the presence of a base (typically, tributylamine) in a refluxing organic solvent such as xylene.

The saturated telechelic polymer is then deprotected using a reaction scheme specific to the protecting group used. For example, if the protecting group is an acetate, the polymer is hydrolyzed under conditions effective to convert the acetate end groups to hydroxyl groups. Typically, such conditions include the use of sodium methoxide in an organic solvent (e.g., methanol) at a temperature of about 0°C to about 100°C (preferably, about 0°C to about 25°C) and a time of about 1 minute to about 1 day (preferably, about 4 hours to about 1 day).

Alternatively, the unsaturated telechelic polymer could be deprotected prior to being hydrogenated to the saturated telechelic polymer.

The invention has been described with reference to various specific and preferred embodiments and will be further described by reference to the following detailed examples.

### Examples

All glassware was dried prior to use. All reactions were performed in a nitrogen or argon atmosphere unless otherwise noted. Hexanes, chloroform, sodium hydroxide, AMBERLITE IRC-718 ion exchange resin, ALIQUOT 336, anhydrous magnesium sulfate, silica gel, activated neutral alumina, toluene, dibutyltin dilaurate, tetrahydrofuran (THF), dioxane, and 10% palladium on activated carbon are all available from Sigma-Aldrich, Milwaukee, WI. Prior to use, the AMBERLITE IRC-718 ion exchange resin beads were dried using a rotary evaporator. Tricyclohexylphosphine[1,3-bis(2,4,6-trimethylphenyl)-4,5-dihydroimidazol-2-ylidene][benzylidene]ruthenium(IV) dichloride (Grubbs' imidazolium metathesis catalyst) was purchased from Strem Chemicals Inc., Newburyport, MA, and stored at -30°C in an argon atmosphere glovebox until used. The source of 10-undecen-1-yl acetate was Bedoukian Research, Incorporated, Danbury, CT. The source of 1,4-butanediol (BDO) was Mitsubishi Chemical, America, Inc., White Plains, NY. The source of solid, flaked 4,4'-methylenebis(phenylisocyanate) (MDI) was Bayer Corporation, Pittsburgh, PA, and sold as fused MONDUR M. The temperatures reported for metathesis reactions were measured using a thermocouple placed between the flask and the heating mantle.

6,6-Dimethyl-1,10-undecadiene was synthesized as described in Example 1 of U.S. Application Publication No. 2003-0125499, published on July 3, 2003.

Synthesis of 7,7-Diethyl-7-silyl-1,12-tridecadiene: One hundred grams of 1,5-hexadiene (Aldrich) was placed in a 500-milliliter round-bottomed three-neck flask. The flask was outfitted with a magnetic stirbar, heating mantle, water-cooled condenser, thermocouple, and addition funnel. The flask was heated with stirring. Meanwhile, the addition funnel was charged with 25 milliliters diethylsilane (Aldrich) and 200 grams 1,5-hexadiene. Two milliliters of a platinum-divinyltetramethyldisiloxane complex in xylene (2-3% Pt) (United Chemical Technologies, Bristol, PA) was added to the flask. The mixture in the addition funnel was added dropwise when the contents of the flask reached 40°C. A small exotherm was observed. After the addition was complete, the mixture was stirred overnight at 40°C. The reaction mixture was then transferred to a one-liter single-neck round-bottomed flask and the excess 1,5-hexadiene was removed using a rotary evaporator. The contents of the flask were then diluted with five volumes of hexanes and dried AMBERLITE IRC-718 ion exchange resin beads were added to sequester the platinum. The reaction mixture was then further purified by passage through a 1.5-cm diameter chromatography column to which had been added about 15 cm of silica gel, followed by 15 centimeters (cm) of activated neutral alumina. Additional hexane was used to elute the product until a sample of eluent evaporated on a watchglass left no residue.

### Example 1: Synthesis of an unsaturated copolymer containing gem-dimethyl and diethylsilane moieties

Into a 250 milliliter (mL) round-bottomed flask, 16.9 grams (g) 6,6-dimethyl-1,10-undecadiene and 94.72 g 7,7-diethyl-7-silyl-1,12-tridecadiene were added. The solution was sparged with nitrogen for 30 minutes. The flask was transferred to a nitrogen-atmosphere glovebox and 0.21 g of tricyclohexylphosphine[1,3-bis(2,4,6-trimethylphenyl)-4,5-dihydroimidazol-2-ylidene][benzylidene]ruthenium(IV) dichloride (Grubbs' imidazolium catalyst) was added. The light red solution was magnetically stirred and vacuum was applied. The solution began bubbling as ethylene was released. The pressure within the flask stabilized at 65 Pascals (Pa), and the temperature of the reaction was 33°C. After 88 hours, the solution was brown in color, no bubbles were observed, and the pressure within the flask was 7 Pa. The temperature was raised to 60°C and upon heating, the pressure rose to 22 Pa. The pressure dropped to 6 Pa after 137 hours of reaction time. The reaction flask was removed from the glovebox and 150 mL hexanes and 17 g dried AMBERLITE IRC-718 ion exchange resin was added. The mixture was magnetically stirred slowly until the dark brown solution became lighter in color. An additional 12 g of the dried AMBERLITE ion exchange resin was added. The solution became pale orange in color. The ion exchange resin was removed by filtration using a Buechner funnel with Whatman Number 2 filter paper. Addition hexanes were used to transfer the product from the Erlenmeyer flask to a one-liter round-bottomed flask. Most of the hexanes were removed by rotary evaporation. The product was passed through a fritted glass column in which had been placed sequentially sand (0.5 cm), silica (4 cm), activated neutral alumina (4 cm), and an additional layer of sand (0.5 cm). The pale orange product became clear and colorless upon elution through the column.

To build a higher molecular weight polymer, the product was treated a second time with catalyst. Inside the nitrogen-atmosphere glovebox, 0.27 g Grubbs' imidazolium catalyst was added to the flask. Bubbling was observed and the pressure was 933 Pa. The reaction temperature was 33°C for 16 hours and then was increased to 60°C. The brown solution had become more viscous and large bubbles were forming. After 96 hours of reacting at 60°C, bubbles were still observed and the pressure was 2.5 Pa. After an additional 24 hours, no more bubbles were observed and the flask was removed from the glovebox. Hexanes (200 mL) and 26 g dried AMBERLITE IRC-718 ion exchange resin were added to the brown polymer. The mixture was stirred for three hours and the color changed from dark to light brown. An additional 13 g of the dried ion exchange resin was added and the solution was stirred until it became lighter in color. The Amberlite was filtered using a Buechner funnel with Whatman Number 40 filter paper. The solution was further purified by passage through a column as described in the previous step. The hexanes were removed by rotary evaporation. The final yield was 77.82 g. The NMR data indicated that there were 18.3 repeat groups on average, and the molecular weight was calculated to be 4355 g/mol.

The signals observed by proton NMR were: δ 5.8, 5.5-5.3, 5.0, 2.0, 1.65, 1.4-1.2, 0.9, 0.8, 0.6-0.4 ppm. The signals observed by ¹³C NMR were: δ130, 36.9, 34.1, 33.9, 32.3, 24.2, 23.6, 23.4, 11.7, 7.5, and 3.8 ppm.

The absorbances observed by FTIR were: 2951, 2874, 2852, 1640, 1457, 1414, 1377, 1340, 1304, 1235, 1168, 1013, 965, 909, 850, 753, and 720 cm⁻¹.

### Example 2: Synthesis of an acetoxytelechelic copolymer containing gem-dimethyl and diethylsilane moieties

A portion of the unsaturated copolymer (54.07 g) of Example 1 was transferred to a 500 mL single-neck, round-bottomed flask. To this flask, 25.5 g 1,20-diacetoxyeicosa-10-ene were added. In a nitrogen-atmosphere glovebox, the mixture was heated to 60°C and stirred until a homogenous solution formed. The solution became clear and colorless, at which time 0.13 g Grubbs' imidazolium catalyst was added. The solution was magnetically stirred and vacuum was applied. The pressure within the flask decreased to 104 Pa and vigorous bubbling was observed. After 18.5 hours, the pressure was 2 Pa and bubbles were no longer observed. The flask was removed from the glovebox and 120 mL hexanes and 26 g dried AMBERLITE IRC-718 were added. The solution was stirred until it became lighter in color. The AMBERLITE was filtered using a Buechner funnel with Whatman Number 40 filter paper. The solution was sent through a column set up as described in Example 1. After elution through the column, the hexanes were removed from the polymer solution by rotary-evaporation. This provided 73.54 g of acetoxytelechelic copolymer, a yellow, slightly viscous liquid.

The signals observed by proton NMR were: δ 5.5-5.3, 4.05 (t), 2.05 (s), 2.1-1.9, 1.65, 1.4-1.2, 0.9, 0.8, 0.6-0.4 ppm. The signals observed by ¹³C NMR were: δ171.3, 130, 64.7, 33.9, 32.7, 32.3, 29.6, 29.4, 28.7, 26.0, 23.4, 11.7, 7.5, and 3.8 ppm.

The absorbances observed by FTIR were: 2951, 2874, 2852, 1744, 1458, 1415, 1386, 1364, 1237, 1169, 1036, 1014, 966, 851, 753, 721, and 605 cm⁻¹.

### Example 3: Synthesis of an unsaturated hydroxytelechelic copolymer containing gem-dimethyl and diethylsilane moieties

To a one-liter round-bottomed flask containing 73.54 g of the acetoxy-functional copolymer of Example 2, 160 mL hexanes, 83 g of 50% NaOH solution and 4.43 g ALIQUOT 336 were added. The solution was magnetically stirred and brought to reflux. After 2.5 hours, the deprotection was complete, as observed by FTIR. The solution was transferred to a one liter separatory funnel. The organic layer was rinsed with water until the pH was neutral. A total of 3 Liters of wash water was used before a neutral pH was obtained. Approximately 100 mL chloroform was added to the separatory funnel to help dissipate the emulsion that had formed. The organic layer was transferred to a one liter Erlenmeyer flask and magnesium sulfate was added to the flask to dry the solution. The anhydrous magnesium sulfate was filtered using a Buechner funnel and Whatman Number 2 filter paper. The solution was then transferred to a one liter round-bottomed flask and the hexanes were removed by rotary-evaporation. The product was transferred to a 500 mL round-bottomed flask, using a small amount of hexanes to ensure complete transfer, which were again removed by rotary-evaporation. The result was 65.71 g hazy brown unsaturated hydroxytelechelic copolymer containing *gem*-dimethyl and diethylsilane moieties. The molecular weight of the diol was calculated by NMR to be 1322 g/mol.

The signals observed by proton NMR were: δ 5.4, 3.6 (t), 2.05 (s), 2.0, 1.53, 1.3-1.1, 0.9, 0.8, 0.6-0.4 ppm. The signals observed by ¹³C NMR were: δ130, 63.2, 33.9, 32.9, 32.3, 29.6, 29.5, 25.8, 23.4, 11.7, 11.6, 11.4, 7.5, and 3.8 ppm.

The absorbances observed by FTIR were: 3322, 2951, 2873, 2853, 1457, 1414, 1377, 1341, 1235, 1168, 1057, 1014, 965, 852, 753, 721, and 586 cm⁻¹.

### Example 4: Synthesis of a saturated hydroxytelechelic copolymer containing gem-dimethyl and diethylsilane moieties

The polymer of Example 3 was hydrogenated in a Parr pressure reactor. The sample was dissolved in toluene sufficient to obtain a 10% solids solution. The hydrogenation was run at 4.14 MPa and 60°C using 10% Pd/C as catalyst to obtain the fully saturated hydroxytelechelic copolymer. The hydrogenation was continued until no further uptake of hydrogen was observed. The result was 58.98 g of pale yellow, fully saturated, copolymer.

The signals observed by proton NMR were: δ 3.6 (t), 1.55, 1.2, 0.9, 0.8, 0.5 ppm. The signals observed by ¹³C NMR were: δ 63.1, 34.0, 32.9, 29.7, 29.4, 25.8, 24.1, 23.9, 11.8, 7.6, and 3.8 ppm.

The absorbances observed by FTIR were: 3336, 2951, 2873, 2853, 1465, 1414, 1377, 1364, 1339, 1306, 1235, 1179, 1057, 1014, 970, 755, 718, and 586 cm⁻¹.

### Example 5: Polyurethane synthesis using the saturated hydroxytelechelic copolymer

Inside a nitrogen-atmosphere glovebox, 5.49 g of the saturated hydroxytelechelic copolymer of Example 4, 3.61 g MDI and 59.43 g solvent (1:1 THF:dioxane containing .009 % dibutyltin dilaurate catalyst) were added to a dry 3-neck round-bottomed flask. The flask was outfitted with a heating mantle, thermocouple connected to a temperature controller, and a condenser. The solution was magnetically stirred and allowed to react at room temperature. After 2.5 hours, a sample was taken for FTIR analysis. The disappearance of the broad hydroxyl peak above 3000 cm⁻¹ showed that the diol had completely reacted with the MDI. New peaks at 3329 and 1701 cm⁻¹ confirmed the formation of urethane bonds. As expected, a large excess of isocyanate functionality remained, as indicated by a strong peak at 2275 cm⁻¹. To the flask, 0.89 g 1,4-butanediol (BDO) was added by syringe. The temperature of the reaction was slowly increased to 50°C. The reaction was allowed to proceed for 17 hours and was monitored by FTIR. More BDO was added in 0.04 g increments as necessary until the isocyanate peak at 2275 cm⁻¹ was very small. A total of 0.12 g more BDO was added over a period of 6 hours after the initial 17 hours of reaction time. After the last aliquot of BDO was added, the solution was held at 50°C for an additional 18 hours, at which point the isocyanate peak was extremely small.

The absorbances of the urethane observed by FTIR were: 3325, 3192, 3124, 3040, 2900, 2873, 2853, 2279, 1703, 1597, 1533, 1465, 1414, 1311, 1231, 1110, 1074, 1017, 962, 915, 849, 816, 769, 717, 668, 610, 586, 510 cm⁻¹.

The solution was allowed to cool to room temperature and the polyurethane was precipitated into methanol in a Waring blender. The polyurethane was dried under vacuum at 70°C for 18 hours. The result was 8.99 g of white, stringy, fluffy precipitate. The polyurethane was pressed into a 0.25 mm thick film at 200°C. The film was clear with a very slight yellow tint. The film was cut into ASTM D638-5 tensile test specimens for tensile strength testing. The initial speed of the pull was 12.7 cm/minute and the crosshead speed was 12.7 cm/minute. Seven samples were tested and the results were averaged. The percent elongation at break was 146.4%. The Young's modulus was 68.4 MPa. The ultimate tensile strength was 17.6 MPa.

## Claims

1. A segmented polymer comprising a soft segment comprising a group of the formula:
-[-(R¹)ₙ-(-Z-(R₂)ₘ-)ₚ-(-Si(R)₂-Vᵣ-)ₛ-]_{q}-
wherein:
n = 0 or 1;
m = 0 or 1;
p = 1-100,000;
r = 0-100,000;
s = 1-100,000;
q = 1-100,000;
R¹ and R² are each independently a saturated or unsaturated aliphatic group, an aromatic group, or combinations thereof, optionally including heteroatoms;
Z is -C(R³)₂- wherein each R³ is independently a saturated or unsaturated aliphatic group, an aromatic group, or combinations thereof, optionally including heteroatoms, wherein the two R³ groups within -C(R³)₂- can be optionally joined to form a ring;
each R is independently a saturated or unsaturated aliphatic group, an aromatic group, or combinations thereof, optionally including heteroatoms; and
V is -O-Si(R)₂- or R¹;
with the proviso that the polymer is free of carbonate linkages.

2. The polymer of claim 1 wherein p = 1-5000.

3. The polymer of claim 1 wherein p = 2-12.

4. The polymer of claim 1 wherein R¹ and R² are each independently a straight chain alkylene group, an arylene group, or combinations thereof.

5. The polymer of claim 4 wherein R¹ and R² are each independently a straight chain alkylene group.

6. The polymer of claim 1 wherein R¹ and R² are each independently groups containing 2 to 20 carbon atoms.

7. The polymer of claim 1 wherein each R³ is independently a straight chain alkyl group, an aryl group, or combinations thereof, optionally including heteroatoms.

8. The polymer of claim 7 wherein each R³ is independently a straight chain alkyl group, optionally including heteroatoms.

9. The polymer of claim 8 wherein each R³ is independently a straight chain alkyl group containing 1 to 20 carbon atoms.

10. The polymer of claim 1 which comprises a urethane group, a urea group, or combinations thereof.

11. The polymer of claim 10 wherein p = 1-100.

12. The polymer of claim 11 wherein p = 2-12.

13. The polymer of claim 10 which is a segmented polyurethane.

14. The polymer of claim 10 which is a biomaterial.

15. The polymer of claim 14 which is substantially free of ether, ester, and carbonate linkages.

16. The polymer of claim 1 wherein said soft segment is prepared from a compound of the formula:
Y-[-(R¹)ₙ-(-Z-(R²)ₘ-)ₚ-(-Si(R)₂-Vᵣ-)ₛ-]_{q}-R⁵-Y
wherein:
each Y is independently OH or NR⁴H;
n = 0 or 1;
m = 0 or 1;
p = 1-100,000;
r = 0-100,000;
s = 1-100,000;
q = 1-100,000;
R¹, R², and R⁵ are each independently a saturated or unsaturated aliphatic group, an aromatic group, or combinations thereof, optionally including heteroatoms;
Z is -C(R³)₂- wherein each R³ is independently a saturated or unsaturated aliphatic group, an aromatic group, or combinations thereof, optionally including heteroatoms, wherein the two R³ groups within -C(R³)₂- can be optionally joined to form a ring;
each R is independently a saturated or unsaturated aliphatic group, an aromatic group, or combinations thereof, optionally including heteroatoms;
each R⁴ is independently H or a saturated or unsaturated aliphatic group, an aromatic group, or combinations thereof; and
V is -O-Si(R)₂- or R¹.

17. The polymer of claim 16 wherein p = 1-100.

18. The polymer of claim 17 wherein p = 2-12.

19. The polymer of claim 16 wherein the number average molecular weight of the compound of the formula
Y-[-(R¹)ₙ-(-Z-(R²)ₘ-)ₚ-(-Si(R)₂-Vᵣ-)ₛ-]_{q}-R⁵-Y
is no greater than about 100,000 grams/mole.

20. The polymer of claim 16 wherein R¹ and R² are each independently a straight chain alkylene group, an arylene group, or combinations thereof.

21. The polymer of claim 20 wherein R¹ and R² are each independently groups containing up to 100 carbon atoms.

22. The polymer of claim 21 wherein R¹ and R² are each independently groups containing up to 20 carbon atoms.

23. The polymer of claim 22 wherein R¹ and R² are each independently groups containing 2 to 20 carbon atoms.

24. The polymer of claim 16 wherein each R² includes at least two carbon atoms.

25. The polymer of claim 16 wherein each R³ is independently a straight chain alkyl group, an aryl group, or combinations thereof, optionally including heteroatoms.

26. The polymer of claim 25 wherein each R³ is independently a straight chain alkyl group containing 1 to 20 carbon atoms.

27. The polymer of claim 16 wherein each Y is OH.

28. The polymer of claim 16 wherein each R⁴ is independently H or a straight chain alkyl group.

29. The polymer of any preceding claim which is linear, branched, or crosslinked.

30. A medical device comprising a polymer as claimed in any preceding claim.

31. A method of making a segmented polymer comprising a soft segment comprising a group of the formula:
-[-(R¹)ₙ-(-Z-(R²)ₘ-)ₚ-(-Si(R)₂-Vᵣ-)ₛ-]_{q}-
the method comprising combining an organic compound containing two or more groups capable of reacting with hydroxyl or amine groups with a polymeric starting compound of the formula:
Y-[-(R¹)ₙ-(-Z-(R²)ₘ-)ₚ-(-Si(R)₂-Vᵣ-)ₛ-]_{q}-R⁵-Y
wherein:
each Y is independently OH or NR⁴H;
n = 0 or 1;
m = 0 or 1;
p = 1-100,000;
r = 0-100,000;
s = 1-100,000;
q = 1-100,000;
R¹, R², and R⁵ are each independently a saturated or unsaturated aliphatic group, an aromatic group, or combinations thereof, optionally including heteroatoms;
Z is -C(R³)₂- wherein each R³ is independently a saturated or unsaturated aliphatic group, an aromatic group, or combinations thereof, optionally including heteroatoms, wherein the two R³ groups within -C(R³)₂- can be optionally joined to form a ring;
each R is independently a saturated or unsaturated aliphatic group, an aromatic group, or combinations thereof, optionally including heteroatoms;
each R⁴ is independently H or a saturated or unsaturated aliphatic group, an aromatic group, or combinations thereof; and
V is -O-Si(R)₂- or R¹;
with the proviso that the polymer is free of carbonate linkages.

32. A method as claimed in claim 31 wherein the polymeric starting compound is prepared by a method comprising combining monomers of Formula II or Formula III
R¹⁰HC=CH-(R¹¹)_{r'}-(-Si(R)₂-Vᵣ-)ₛ-(R¹²)_{s'}-CH=CHR¹³ (II)
R¹⁰HC=CH-(R¹¹)_{r'}-Z-(R¹²)_{s'}-CH=CHR¹³ (III)
wherein:
r, s, V, Z, and R are as defined in claim 31;
r' = 0 or 1;
s' = 0 or 1;
R¹⁰ and R¹³ are each independently hydrogen or straight chain, branched, or cyclic alkyl groups containing up to 6 carbon atoms; and
R¹¹ and R¹² are each independently a saturated aliphatic group, an aromatic group, or combinations thereof;
with an alkene metathesis catalyst and optionally applying a vacuum.

## Patentansprüche

1. Segmentiertes Polymer, umfassend ein Weichsegment, umfassend eine Gruppe der Formel
**-[-(R¹)ₙ-Z-(R²)ₘ-)ₚ-(-Si(R)₂-Vᵣ-)ₛ-]_{q}-**
wobei
n = 0 oder 1;
m = 0 oder 1;
p = 1-100.000;
r = 0-100.000;
s = 1-100.000;
q = 1-100.000;
R¹ und R² jeweils unabhängig für eine gesättigte oder ungesättigte aliphatische Gruppe, eine aromatische Gruppe oder Kombinationen davon stehen, die wahlweise Heteroatome einschließen;
Z für -C(R³)₂- steht, wobei jedes R³ unabhängig für eine gesättigte oder ungesättigte aliphatische Gruppe, eine aromatische Gruppe oder Kombinationen davon steht, die wahlweise Heteroatome einschließen, wobei die beiden R³-Gruppen in -C(R³)₂- wahlweise unter Bildung eines Rings verbunden sein können;
jedes R unabhängig für eine gesättigte oder ungesättigte aliphatische Gruppe, eine aromatische Gruppe oder Kombinationen davon steht, die wahlweise Heteroatome einschließen; und
V für -O-Si(R)₂- oder R¹ steht;
mit der Maßgabe, dass das Polymer frei von Carbonatbindungen ist.

2. Polymer nach Anspruch 1, wobei p = 1-5000.

3. Polymer nach Anspruch 1, wobei p = 2-12.

4. Polymer nach Anspruch 1, wobei R¹ und R² jeweils unabhängig für eine geradkettige Alkylengruppe, eine Arylengruppe oder Kombinationen davon stehen.

5. Polymer nach Anspruch 4, wobei R¹ und R² jeweils unabhängig für eine geradkettige Alkylengruppe stehen.

6. Polymer nach Anspruch 1, wobei R¹ und R² jeweils unabhängig für Gruppen stehen, die 2 bis 20 Kohlenstoffatome enthalten.

7. Polymer nach Anspruch 1, wobei jedes R³ unabhängig für eine geradkettige Alkylgruppe, eine Arylgruppe oder Kombinationen davon steht, die wahlweise Heteroatome einschließen.

8. Polymer nach Anspruch 7, wobei jedes R³ unabhängig für eine geradkettige Alkylgruppe steht, die wahlweise Heteroatome einschließt.

9. Polymer nach Anspruch 8, wobei jedes R³ unabhängig für eine geradkettige Alkylgruppe steht, die 1 bis 20 Kohlenstoffatome enthält.

10. Polymer nach Anspruch 1, das eine Urethangruppe, eine Harnstoffgruppe oder Kombinationen davon umfasst.

11. Polymer nach Anspruch 10, wobei p = 1-100.

12. Polymer nach Anspruch 11, wobei p = 2-12.

13. Polymer nach Anspruch 10, bei welchem es sich um ein segmentiertes Polyurethan handelt.

14. Polymer nach Anspruch 10, bei welchem es sich um ein Biomaterial handelt.

15. Polymer nach Anspruch 14, das im Wesentlichen frei von Ether-, Ester- und Carbonatbindungen ist.

16. Polymer nach Anspruch 1, wobei das Weichsegment aus einer Verbindung der folgenden Formel hergestellt ist:
**Y-[-(R¹)ₙ-(-Z-(R²)ₘ-)ₚ-(-Si(R)₂-Vᵣ-)ₛ-]_{q}-R⁵-Y**
wobei
jedes Y unabhängig für OH oder NR⁴H steht;
n = 0 oder 1;
m = 0 oder 1;
p = 1-100.000;
r = 0-100.000;
s = 1-100.000;
q = 1-100.000;
R¹, R² und R⁵ jeweils unabhängig für eine gesättigte oder ungesättigte aliphatische Gruppe, eine aromatische Gruppe oder Kombinationen davon stehen, die wahlweise Heteroatome einschließen;
Z für -C(R³)₂- steht, wobei jedes R³ unabhängig für eine gesättigte oder ungesättigte aliphatische Gruppe, eine aromatische Gruppe oder Kombinationen davon steht, die wahlweise Heteroatome einschließen, wobei die beiden R³-Gruppen in -C(R³)₂- wahlweise unter Bildung eines Rings verbunden sein können;
jedes R unabhängig für eine gesättigte oder ungesättigte aliphatische Gruppe, eine aromatische Gruppe oder Kombinationen davon steht, die wahlweise Heteroatome einschließen;
jedes R⁴ unabhängig für H oder eine gesättigte oder ungesättigte aliphatische Gruppe, eine aromatische Gruppe oder Kombinationen davon steht; und
V für -O-Si(R)₂- oder R¹ steht.

17. Polymer nach Anspruch 16, wobei p = 1-100.

18. Polymer nach Anspruch 17, wobei p = 2-12.

19. Polymer nach Anspruch 16, wobei das Zahlenmittel des Molekulargewichts der Verbindung der Formel
**Y-[-(R¹)ₙ-(-Z-(R²)ₘ-)ₚ-(-Si(R)₂-Vᵣ-)ₛ-]_{q}-R⁵-Y**
nicht mehr als etwa 100.000 g/mol beträgt.

20. Polymer nach Anspruch 16, wobei R¹ und R² jeweils unabhängig für eine geradkettige Alkylengruppe, eine Arylengruppe oder Kombinationen davon stehen.

21. Polymer nach Anspruch 20, wobei R¹ und R² jeweils unabhängig für Gruppen stehen, die bis zu 100 Kohlenstoffatome enthalten.

22. Polymer nach Anspruch 21, wobei R¹ und R² jeweils unabhängig für Gruppen stehen, die bis zu 20 Kohlenstoffatome enthalten.

23. Polymer nach Anspruch 22, wobei R¹ und R² jeweils unabhängig für Gruppen stehen, die 2 bis 20 Kohlenstoffatome enthalten.

24. Polymer nach Anspruch 16, wobei jedes R² mindestens zwei Kohlenstoffatome einschließt.

25. Polymer nach Anspruch 16, wobei jedes R³ unabhängig für eine geradkettige Alkylgruppe, eine Arylgruppe oder Kombinationen davon steht, die wahlweise Heteroatome einschließen.

26. Polymer nach Anspruch 25, wobei jedes R³ unabhängig für eine geradkettige Alkylgruppe steht, die 1 bis 20 Kohlenstoffatome enthält.

27. Polymer nach Anspruch 16, wobei jedes Y für OH steht.

28. Polymer nach Anspruch 16, wobei jedes R⁴ unabhängig für H oder eine geradkettige Alkylgruppe steht.

29. Polymer nach einem der vorangehenden Ansprüche, das linear, verzweigt oder vernetzt ist.

30. Medizinische Vorrichtung, die ein Polymer nach einem der vorangehenden Ansprüche umfasst.

31. Verfahren zur Herstellung eines segmentierten Polymers, das ein Weichsegment umfasst, das eine Gruppe der folgenden Formel umfasst
**-[-(R¹)ₙ-(-Z-(R²)ₘ-)ₚ-(-Si(R)₂-Vᵣ-)ₛ-]_{q}-**
wobei das Verfahren das Kombinieren einer organischen Verbindung, die zwei oder mehr Gruppen enthält, die in der Lage sind, mit Hydroxy- oder Amingruppen zu reagieren, mit einer polymeren Ausgangsverbindung der Formel
**Y-[-(R¹)ₙ-(-Z-(R²)ₘ-)ₚ-(-Si(R)₂-Vᵣ-)ₛ-]_{q}-R⁵-Y**
wobei
jedes Y unabhängig für OH oder NR⁴H steht;
n = 0 oder 1;
m = 0 oder 1;
p = 1-100.000;
r = 0-100.000;
s = 1-100.000;
q = 1-100.000;
R¹, R² und R⁵ jeweils unabhängig für eine gesättigte oder ungesättigte aliphatische Gruppe, eine aromatische Gruppe oder Kombinationen davon stehen, die wahlweise Heteroatome einschließen;
Z für -C(R³)₂- steht, wobei jedes R³ unabhängig für eine gesättigte oder ungesättigte aliphatische Gruppe, eine aromatische Gruppe oder Kombinationen davon steht, die wahlweise Heteroatome einschließen, wobei die beiden R³-Gruppen in -C(R³)₂- wahlweise unter Bildung eines Rings verbunden sein können;
jedes R unabhängig für eine gesättigte oder ungesättigte aliphatische Gruppe, eine aromatische Gruppe oder Kombinationen davon steht, die wahlweise Heteroatome einschließen;
jedes R⁴ unabhängig für H oder eine gesättigte oder ungesättigte aliphatische Gruppe, eine aromatische Gruppe oder Kombinationen davon steht; und
V für -O-Si(R)₂- oder R¹ steht;
mit der Maßgabe, dass das Polymer frei von Carbonatbindungen ist,
umfasst.

32. Verfahren nach Anspruch 31, wobei die polymere Ausgangsverbindung durch ein Verfahren hergestellt wird, das das Kombinieren von Monomeren der Formel II oder Formel III
**R¹⁰HC=CH-(R¹¹)_{r'}-(-Si(R)₂-Vᵣ-)ₛ-(R¹²)_{s'}-CH-CHR¹³** **(II)**
**R¹⁰HC=CH-(R¹¹)_{r'}-Z-(R¹²)_{s'}-CH=CHR¹³** **(III)**
wobei
r, s, V, Z und R wie in Anspruch 31 definiert sind;
r' = 0 oder 1;
s' = 0 oder 1;
R¹⁰ und R¹³ jeweils unabhängig für Wasserstoff oder geradkettige, verzweigte oder cyclische Alkylgruppen stehen, die bis zu 6 Kohlenstoffatome enthalten; und
R¹¹ und R¹² jeweils unabhängig für eine gesättigte aliphatische Gruppe, eine aromatische Gruppe oder Kombinationen davon stehen;
mit einem Alkenmetathesekatalysator und wahlweise das Anlegen von Vakuums umfasst.

## Revendications

1. Polymère segmenté comprenant un segment mou comprenant un groupe de la formule :
**-[-(R¹)ₙ-(-Z-(R²)ₘ-)ₚ-(-Si(R)₂-Vᵣ-)ₛ-]_{q}-**
dans laquelle :
- n = 0 ou 1 ;
- m = 0 ou 1 ;
- p = 1-100000 ;
- r = 0-100000 ;
- s = 1-100000 ;
- q = 1-100000 ;
- R¹ et R² sont chacun indépendamment un groupe aliphatique saturé ou insaturé, un groupe aromatique ou des combinaisons de ceux-ci, contenant facultativement des hétéroatomes ;
- Z est -C(R³)₂- où chaque R³ est indépendamment un groupe aliphatique saturé ou insaturé, un groupe aromatique ou des combinaisons de ceux-ci, contenant facultativement des hétéroatomes, les deux groupes R³ dans -C(R³)₂- pouvant être facultativement réunis pour former un cycle ;
- chaque R est indépendamment un groupe aliphatique saturé ou insaturé, un groupe aromatique ou des combinaisons de ceux-ci, contenant facultativement des hétéroatomes ; et
- V représente -O-Si(R)₂- ou R¹ ;
à la condition que le polymère soit exempt de liaisons carbonate.

2. Polymère selon la revendication 1, dans lequel p = 1-5000.

3. Polymère selon la revendication 1, dans lequel p = 2-12.

4. Polymère selon la revendication 1, dans lequel R¹ et R² sont chacun indépendamment un groupe alkylène à chaîne droite, un groupe arylène ou des combinaisons de ceux-ci.

5. Polymère selon la revendication 4, dans lequel R¹ et R² sont chacun indépendamment un groupe alkylène à chaîne droite.

6. Polymère selon la revendication 1, dans lequel R¹ et R² sont chacun indépendamment des groupes contenant 2 à 20 atomes de carbone

7. Polymère selon la revendication 1, dans lequel chaque R³ est indépendamment un groupe alkyle à chaîne droite, un groupe aryle ou des combinaisons de ceux-ci, contenant facultativement des hétéroatomes.

8. Polymère selon la revendication 7, dans lequel chaque R³ est indépendamment un groupe alkyle à chaîne droite, contenant facultativement des hétéroatomes.

9. Polymère selon la revendication 8, dans lequel chaque R³ est indépendamment un groupe alkyle à chaîne droite contenant 1 à 20 atomes de carbone.

10. Polymère selon la revendication 1, qui comprend un groupe uréthane, un groupe urée ou des combinaisons de ceux-ci.

11. Polymère selon la revendication 10, dans lequel p = 1-100.

12. Polymère selon la revendication 11, dans lequel p = 2-12.

13. Polymère selon la revendication 10, qui est un polyuréthane segmenté.

14. Polymère selon la revendication 10, qui est un bio-matériau.

15. Polymère selon la revendication 14 qui est sensiblement exempt de liaisons éther, ester et carbonate.

16. Polymère selon la revendication 1, dans lequel ledit segment mou est préparé à partir d'un composé de la formule :
**Y-[-(R¹)ₙ-(-Z-(R²)ₘ-)ₚ-(-Si(R)₂-Vᵣ-)ₛ-]_{q}**-**R⁵-Y**
dans laquelle :
- chaque Y est indépendamment OH ou NR⁴H ;
- n = 0 ou 1 ;
- m = 0 ou 1 ;
- p = 1-100000 ;
- r = 0-100000 ;
- s = 1-100000 ;
- q = 1-100000 ;
- R¹, R² et R⁵ sont chacun indépendamment un groupe aliphatique saturé ou insaturé, un groupe aromatique ou des combinaisons de ceux-ci, contenant facultativement des hétéroatomes ;
- Z est -C(R³)₂- où chaque R³ est indépendamment un groupe aliphatique saturé ou insaturé, un groupe aromatique ou des combinaisons de ceux-ci, contenant facultativement des hétéroatomes, les deux groupes R³ dans -C(R³)₂- pouvant être facultativement réunis pour former un cycle ;
- chaque R est indépendamment un groupe aliphatique saturé ou insaturé, un groupe aromatique ou des combinaisons de ceux-ci, contenant facultativement des hétéroatomes ; et
- chaque R⁴ est indépendamment H ou un groupe aliphatique saturé ou insaturé, un groupe aromatique ou des combinaisons de ceux-ci ; et
- V est -O-Si(R)₂- ou R¹.

17. Polymère selon la revendication 16, dans lequel p = 1-100.

18. Polymère selon la revendication 17, dans lequel p = 2-12.

19. Polymère selon la revendication 16, dans lequel la masse moléculaire moyenne en nombre du composé de la formule
**Y-[-(R¹)ₙ-(-Z-(R²)ₘ-)ₚ-(-Si(R)₂-Vᵣ-)ₛ-]_{q}-R⁵-Y**
n'est pas supérieure à environ 100000 grammes/mole.

20. Polymère selon la revendication 16, dans lequel R¹ et R² sont chacun indépendamment un groupe alkylène à chaîne droite, un groupe arylène ou des combinaisons de ceux-ci.

21. Polymère selon la revendication 20, dans lequel R¹ et R² sont chacun indépendamment des groupes contenant jusqu'à 100 atomes de carbone.

22. Polymère selon la revendication 21, dans lequel R¹ et R² sont chacun indépendamment des groupes contenant jusqu'à 20 atomes de carbone.

23. Polymère selon la revendication 22, dans lequel R¹ et R² sont chacun indépendamment des groupes contenant 2 à 20 atomes de carbone.

24. Polymère selon la revendication 16, dans lequel chaque R² comprend au moins deux atomes de carbone.

25. Polymère selon la revendication 16, dans lequel chaque R³ représente indépendamment un groupe alkyle à chaîne droite, un groupe aryle ou des combinaisons de ceux-ci, contenant facultativement des hétéroatomes.

26. Polymère selon la revendication 25, dans lequel chaque R³ est indépendamment un groupe alkyle à chaîne droite contenant 1 à 20 atomes de carbone.

27. Polymère selon la revendication 16, dans lequel chaque Y est OH.

28. Polymère selon la revendication 16, dans lequel chaque R⁴ est indépendamment H ou un groupe alkyle à chaîne droite.

29. Polymère selon l'une quelconque des revendications précédentes qui est linéaire, ramifié ou réticulé.

30. Dispositif médical comprenant un polymère tel que défini à l'une quelconque des revendications précédentes.

31. Procédé de fabrication d'un polymère segmenté comprenant un segment mou comprenant un groupe de la formule :
**-[-(R¹)ₙ-(-Z-(R²)ₘ-)ₚ-(-Si(R)₂-Vᵣ-)ₛ-]_{q}-**
le procédé comprenant la combinaison d'un composé organique contenant deux groupes ou davantage capables de réagir avec des groupes hydroxyle ou amine avec un composé de départ polymère de la formule :
**Y-[-(R¹)ₙ-(-Z-(R²)ₘ-)ₚ-(-Si(R)₂-Vᵣ-)ₛ-]_{q}-R⁵-Y**
dans laquelle :
- chaque Y est indépendamment OH ou NR⁴H ;
- n = 0 ou 1 ;
- m = 0 ou 1 ;
- p = 1-100000 ;
- r = 0-100000 ;
- s = 1-100000 ;
- q = 1-100000 ;
- R¹, R² et R⁵ sont chacun indépendamment un groupe aliphatique saturé ou insaturé, un groupe aromatique ou des combinaisons de ceux-ci, contenant facultativement des hétéroatomes ;
- Z est -C(R³)₂- où chaque R³ est indépendamment un groupe aliphatique saturé ou insaturé, un groupe aromatique ou des combinaisons de ceux-ci, contenant facultativement des hétéroatomes, les deux groupes R³ dans -C(R³)₂- pouvant être facultativement réunis pour former un cycle ;
- chaque R est indépendamment un groupe aliphatique saturé ou insaturé, un groupe aromatique ou des combinaisons de ceux-ci, contenant facultativement des hétéroatomes ;
- chaque R⁴ est indépendamment H ou un groupe aliphatique saturé ou insaturé, un groupe aromatique ou des combinaisons de ceux-ci ; et
- V représente -O-Si(R)₂- ou R¹ ; à la condition que le polymère soit exempt de liaisons carbonate.

32. Procédé selon la revendication 31, dans lequel le composé de départ polymère est préparé par un procédé comprenant la combinaison de monomères de Formule II ou de Formule III
**R¹⁰HC=CH-(R¹¹)_{r'}-(-Si(R)₂-Vᵣ-)ₛ-(R¹²)_{s'}-CH=CHR¹³** **(II)**
**R¹⁰HC=CH-(R¹¹)_{r'}-Z-(R¹²)_{s'}-CH=CHR¹³** **(III)**
où :
- r, s, V, Z et R sont tels que définis à la revendication 31 ;
- r' = 0 ou 1 ;
- s' = 0 ou 1 ;
- R¹⁰ et R¹³ sont chacun indépendamment hydrogène ou des groupes alkyle à chaîne droite, ramifiés ou cycliques contenant jusqu'à 6 atomes de carbone ; et
- R¹¹ et R¹² sont chacun indépendamment un groupe aliphatique saturé, un groupe aromatique ou des combinaisons de ceux-ci,
avec un catalyseur de métathèse d'alcène et facultativement l'application d'un vide.
